# EUROPEAN PATENT APPLICATION

(11) **EP 4 434 496 A1**
(43) Date of publication of application: **25.09.2024**
(21) Application number: 24165321.1
(22) Date of filing: 21.03.2024
(51) Int. Cl.: A61F 2/30, A61F 2/46, A61B 17/72, A61F 2/40, A61F 2/36

(54) **MODULAR IMPLANT SYSTEM**

(30) Priority: 22.03.2023 US 202363453941 P
(71) Applicant: Zimmer, Inc., Warsaw, IN 46580 (US)
(72) Inventor: MILLER, Kevin, Wakarusa (US); LINDSLEY, Katherine B., South Bend (US); ZEARING, Luke Anthony, Ebensburg (US)
(74) Representative: Taor, Simon Edward William

(57) **Abstract**

A modular implant (100) system including a first component (102) and a second component (104). The first component includes a stem (106) adapted to be anchored within a medullary canal of a bone and a mounting boss (108) defining an outer coupling surface (114) and an inner coupling surface (202). The second component includes a collar (116) defining a first coupling surface (118) adapted to engage the outer coupling surface of the mounting boss to secure the second component to the first component.

## Description

### CLAIM OF PRIORITY

This application claims the benefit of U.S. Provisional Patent Application Serial No. 63/453,941, filed on March 22, 2023, the benefit of priority of which is claimed hereby, and which is incorporated by reference herein in its entirety.

### TECHNICAL FIELD

This document pertains generally, but not by way of limitation, to prosthetic implants for use in joint replacement procedures. More particularly, but not by way of limitation, this document pertains to a stemmed implant system including a variety of removeable components adapted to engage one or more bone surfaces of a proximal humerus or a proximal femur.

### BACKGROUND

In a healthy shoulder joint (e.g., the glenohumeral joint), the proximal humerus includes a humeral head which articulates within the glenoid fossa of the scapula, and several other bony landmarks which serve as attachment points for the rotator cuff. Over time, various ailments such as, among others, osteoarthritis, rotator cuff tears, avascular necrosis, humeral fractures, failure of a previously implanted humeral prosthetic, cancer or cancer treatments, infections, traumatic injuries, or bone loss caused by various conditions, can diminish the functionality of the shoulder joint and cause significant pain and discomfort. Such ailments often lead to surgical correction in the form of a shoulder arthroplasty (e.g., a shoulder replacement procedure), during which prosthetic components are implanted into a patient to replace the patient's natural humeral head and glenoid fossa.

### SUMMARY

In an example, a modular implant system can comprise a first component including a stem including a proximal portion and a distal portion, wherein the distal portion is adapted to be anchored within a medullary canal of a bone, and a mounting boss extending from the proximal portion, wherein the mounting boss defines an outer coupling surface and an inner coupling surface. The modular implant system can further comprise a second component including a collar defining a first coupling surface, wherein the first coupling surface is adapted to engage the outer coupling surface of the mounting boss of the stem to secure the second component to the first component.

In an example, a modular implant system can comprise a first component including a stem including a proximal portion and a distal portion, wherein the distal portion is adapted to be anchored within a medullary canal of a bone, and a mounting boss extending from the proximal portion, wherein the mounting boss defines an outer coupling surface and an inner coupling surface. The modular implant system can further comprise a plurality of second components. Each second component of the plurality of second components can include a collar defining a first coupling surface, wherein the first coupling surface is adapted to engage the outer coupling surface of the mounting boss to secure any of the plurality of second components to the first component. Each second component of the plurality of second components can be uniquely sized and shaped relative to other second components of the plurality of second components.

In an example, a method of adapting a modular implant system to a patient can comprise selecting a first component from a plurality of first components, selecting a second component from a plurality of second components, and securing the second component to the first component. The first component can be adapted to be anchored within a medullary canal of a bone of the patient. Selection of the second component from the plurality of second components can be based on a physical characteristic of the bone. The second component can be adapted to engage at least one of a metaphysis or a diaphysis of the bone of the patient.

### BRIEF DESCRIPTION OF THE DRAWINGS

FIG. 1 illustrates an isometric view of an example modular implant system.
FIG. 2A illustrates an isometric view of an example modular implant system including a first revision component.
FIG. 2B illustrates side view of an example second revision component positioned within a proximal humerus.
FIG. 3A illustrates an isometric view of an example second revision component.
FIG. 3B illustrates a rear view of an example second revision component.
FIGS. 4A-4B illustrate isometric views of an example modular implant system including a second revision component.
FIGS. 5A-5B illustrate side views of an example modular implant system including a third revision component.
FIGS. 6A-6B illustrate isometric views of an example modular implant system including a first fracture component.
FIGS. 7A-7B illustrate isometric views of an example modular implant system including a first fracture component.
FIG. 8A illustrates an isometric view of an example second fracture component.
FIG. 8B illustrates a side view of an example second fracture component.
FIGS. 9A-9B illustrate isometric views of an example second fracture component.
FIG. 10 illustrates a partial cross-section of an example modular implant system.
FIG. 11 illustrates a method of adapting a modular implant system to a patient.

In the drawings, which are not necessarily drawn to scale, like numerals may describe similar components in different views. Like numerals having different letter suffixes may represent different instances of similar components. The drawings illustrate generally, by way of example, but not by way of limitation, various embodiments discussed in the present document.

### DETAILED DESCRIPTION

A variety of stemmed prosthetic implants are presently available for use in joint replacement procedures. Such implants generally include a stemmed distal portion that is inserted into a diaphysis of a bone and a proximal portion that receives either a prosthetic humeral head, such as in a total shoulder arthroplasty, or a prosthetic cup for articulating against a ball-shaped head of a prosthetic glenoid implant, such as in a reverse shoulder arthroplasty. However, while many aspects of stemmed implants have evolved over the years, such implants generally include universal or otherwise non-adaptable features adapted to help secure the implant to the proximal humerus or help secure the rotator cuff or displaced bone fragments to the implant. Such features can contribute to undesirable patient outcomes, as the size and shape of a proximal humerus may vary greatly between individual patients.

For example, a universal or non-configurable implant can, in some arthroplasties, provide a significantly limited amount of engagement with, or lead to significant loss of, bone of the proximal humerus. This can lead to stress-shielding of the proximal humerus by inhibiting stress forces generated during joint movement from being effectively transferred into the proximal humerus. Stress-shielding can contribute to diminished post-operative bone ingrowth and reduced long-term bone health and strength. Additionally, in some arthroplasties, a universal or non-configurable implant can make securing displaced bone fragments to the implant in anatomically accurate positions, such as to reconstruct natural insertion points for soft tissue, a difficult operation. In view of the above, a need exists for an improved stemmed implant for use in shoulder, hip, or other arthroplasties.

The present disclosure can help to address the above issues, among others, by providing a modular implant system. For example, the modular implant system can include at least one first component defining an outer coupling surface and at least one second component defining a first coupling surface adapted to engage the outer coupling surface to secure the second component to the first component. The first component can be a stemmed implant adapted to be inserted into a medullary canal of a bone, such as of a humerus or a femur, and can receive a third component adapted to replace a natural humeral head or femoral head. The second component can be an implant adapted to engage a bone, such as a humerus or femur, to transfer stress forces into the bone and help secure soft tissue or displaced bone fragments in anatomically accurate locations. In view of the above, the modular implant system of the present disclosure can provide a number of benefits to both patients and surgeons.

First, the modular implant system can enable a surgeon to utilize a single implant system for many different types of arthroplasties, such as for total shoulder arthroplasties, reverse shoulder arthroplasties, revision shoulder arthroplasties, or fracture correction shoulder arthroplasties. For example, the uniform nature of the outer coupling surface and the first coupling surface can enable a surgeon to pair one of a plurality of different first components with one of a plurality of different second components to find a best fit for the anatomical needs of a specific patient. This can help to provide a high level of convenience for a surgeon, while maintaining a relatively low overall cost for a patient, such as by allowing a surgeon to customize a universally shaped first component with a wide variety of uniquely shaped second components.

Second, the modular implant system can help enable a surgeon to improve both short-term and long-term patient outcomes in various types of arthroplasties. For example, the plurality of second components can allow a surgeon to select a second component based on many different physical characteristics of a patient's bone, such as including, among others, the size and shape of a patient's proximal humerus or femur, an amount of bone that will remain after the bone is resected, or the size, shape, and number of bone fragments displaced from the bone. In view of the above, the modular implant system can provide a higher level of customization relative to existing stemmed implants, such as to, among others, allow the modular implant system to help prevent post operative stress-shielding, more accurately reconstruct natural bony landmarks, and enable a greater degree of bone preservation in at least shoulder or hip arthroplasties.

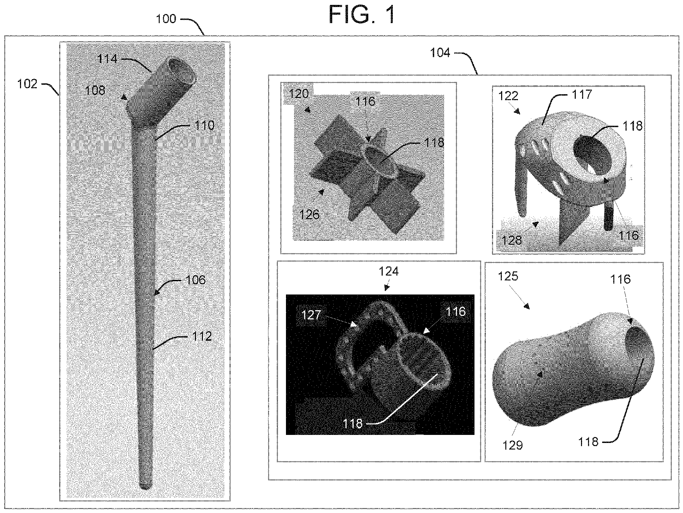

FIG. 1 illustrates an isometric view of an example modular implant system 100. The modular implant system 100 can include a first component 102 and a plurality of second components 104. The first component 102 can include a stem 106 and a mounting boss 108. The stem 106 can define a proximal portion 110 and a distal portion 112. The stem 106 can generally be a frustoconical body defining a tapered cross-section between the proximal portion 110 and the distal portion 112. The distal portion 112 can be adapted to be anchored within a diaphysis of a bone. For example, the distal portion 112 can be sized and shaped to be cemented or press-fit within a medullary canal, such as, but not limited to, a humerus or a femur. In some examples, the stem 106 can be universally sized and shaped.

In other examples, the modular implant system 100 can include a plurality of first components, such as, but not limited to, between two and twenty uniquely configured second revision components to thereby form a standardized range of sizes and shapes (e.g., different longitudinal lengths, outer diameters, or taper angles) for the stem 106. This can enable a surgeon to conveniently select the first component 102 therefrom based on one or more physical characteristics of a patient's bone, such as the natural size and shape of the patient's medullary canal. In such examples, the mounting boss 108 can be uniformly sized and shaped. The mounting boss 108 can generally be a cylindrical, oblong, or tapered body. The mounting boss 108 can define an outer coupling surface 114. The outer coupling surface 114 can be an outer-most surface of the mounting boss 108. Each second component of the plurality of second components 104 can include a collar 116 defining a first coupling surface 118.

The first coupling surface 118 can be an inner-most surface of each second component of the plurality of second components 104. The first coupling surface 118 can be adapted to contact and engage the outer coupling surface 114 of the mounting boss 108 to secure any of the plurality of second components 104 to the first component 102. For example, the first coupling surface 118 can define a female taper and the outer coupling surface 114 can define a male taper, such as to form a Morse taper. In another example, the first coupling surface 118 can define a male taper and the outer coupling surface 114 can define a female taper, such as to form a reverse Morse taper. In an additional example, the first coupling surface 118 can be sized and shaped to engage the outer coupling surface 114 via a press fit.

The plurality of second components 104 can allow a surgeon to customize the modular implant system 100 to the anatomical needs of a wide variety of different patients. The plurality of second components 104 can include a first revision component 120, a second revision component 122, a first fracture component 124, and a second fracture component 125. The first revision component 120 can be adapted, and selected by a surgeon, for arthroplasties where a greater portion of a metaphysis of a bone can be preserved, or will remain, after a resection has been performed. For example, the first revision component 120 can include a plurality of radial fins 126 adapted to extend into cancellous bone of a patient's proximal humerus or femur to transfer stress forces thereinto and secure the modular implant system 100 thereto.

The second revision component 122 can be adapted, and selected by a surgeon, for arthroplasties where a lesser portion or none of a metaphysis of a bone is preserved, or otherwise remains after a resection has been performed. For example, the second revision component 122 can include a head portion 117 sized and shaped to replace various natural bony landmarks and provide numerous attachment points for the rotator cuff to replace various natural rotator cuff insertion points. The second revision component 122 can further include a plurality of longitudinal fins 128 adapted to extend into cancellous bone of a patient's proximal humerus or femur to transfer stress forces thereinto and secure the modular implant system 100 thereto.

The first fracture component 124 and the second fracture component 125 can be adapted, and selected by a surgeon, for arthroplasties where one or more displaced bone fragments, such as of a proximal humerus or proximal femur, can be preserved through fixation to the modular implant system 100. For example, the first fracture component 124 can define a plurality of first apertures 127, and the second fracture component 125 can define a plurality of second apertures 129, each adapted for receiving a suture there through, or a suture anchor therein, to provide a surgeon with a wide variety of options for attaching displaced bone fragments to the modular implant system 100 in anatomically accurate orientations and locations. This can help enable the surgeon to reconstruct various natural bony landmarks, such as providing insertion points, for the rotator cuff or other soft tissues.

In the operation of some examples, a surgeon can obtain imaging data, such as computed topography (hereinafter "CT") data, of a shoulder joint of an individual patient. The imaging data can be reviewed and analyzed to create a surgical plan. In some examples, a surgical plan can be created using a preoperative planning software, such the Signature^{™} ONE Surgical Planning System from Zimmer Biomet, Inc. of Warsaw, Indiana (hereinafter "Zimmer Biomet"). A surgeon can then select the first component 102 and one of the plurality of second components 104 based on the surgical plan. For example, the first component 102 and one of the plurality of second components 104 can be selected based on a number of factors, such as including, but not limited to, the type of arthroplasty to be performed or one or more physical characteristics of the patient's humerus or femur. Finally, the surgeon can assemble the modular implant system 100 by securing the first component 102 to a selected second component of the plurality of second component 104s. In view of the above, the modular implant system 100 can provide a surgeon with many different customization options to help the surgeon find a best fit for the anatomical needs of a wide variety of patients in various types of arthroplasties.

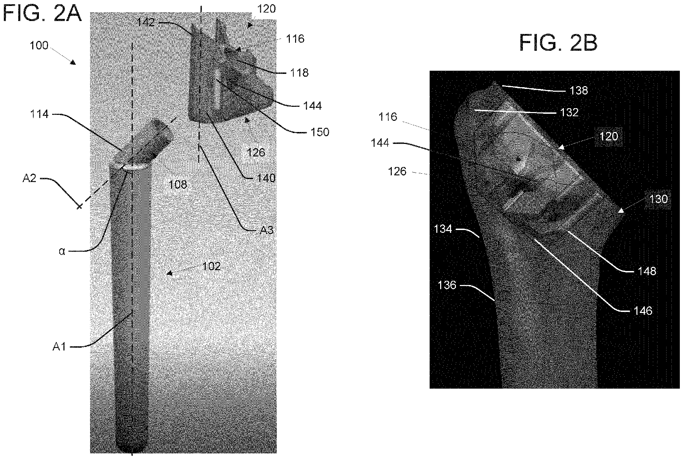

FIG. 2A illustrates an isometric view of an example modular implant system 100 including a first revision component 120. FIG. 2B illustrates a side view of an example first revision component 120 received within a proximal humerus 130. FIG. 2A and FIG. 2B are discussed below concurrently. Also shown in FIG. 2A is a first axis A1, a second axis A2, and a third axis A3. The proximal humerus 130 can include an epiphysis 132 (FIG. 2B), a metaphysis 134 (FIG. 2B), and a diaphysis 136 (FIG. 2B). The proximal humerus 130 can define an outer bone surface 138 (FIG. 2B). The outer bone surface 138 can generally be a planar surface defined or exposed during a resection of a humeral head of the proximal humerus 130.

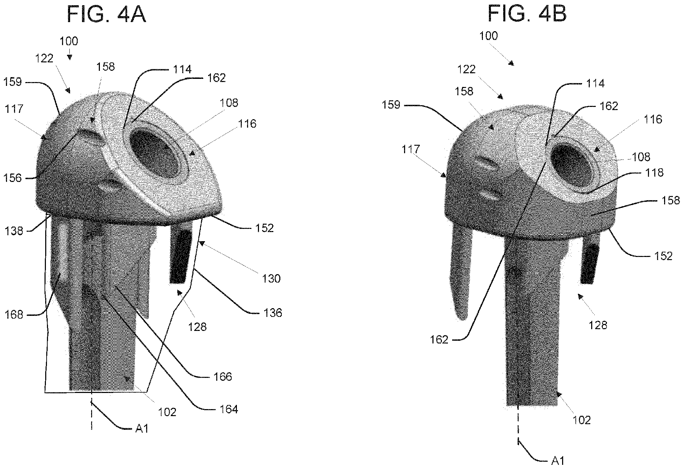

An angle the outer bone surface 138 forms relative to the first axis A1 (FIG. 2A) can vary relatively drastically between patients depending on various factors, such as, but not limited to, the resection angle of a previous resection taken from the proximal humerus 130 or a proximal femur, the extent of bone loss or degradation of the proximal humerus 130 or a proximal femur, or the extent of bone trauma caused by injury or infection. For example, in arthroplasties where a greater portion of the metaphysis 134 is preserved after a resection has been performed, the outer bone surface 138 can extend at, but not limited to, between about 10 degrees and about 40 degrees relative to the first axis A1, such as shown in FIG. 2B. In arthroplasties where a greater portion of the metaphysis 134 is preserved after a resection has been performed, the outer bone surface 138 can extend at, but not limited to, between about 40 degrees and about 90 degrees relative to the first axis A1, such as shown in FIG. 4A.

The first component 102 (FIG. 2A) can define the first axis A1. The first axis A1 can be a longitudinal axis of the stem 106 extending centrally through the proximal portion 110 (FIG. 2A) and the distal portion 112 (FIG. 2A). The second axis A2 can be a longitudinal axis of the mounting boss 108 (FIG. 2A) extending centrally through the mounting boss 108. The mounting boss 108 can extend at various angles relative to the stem 106. For example, an angle α (FIG. 2A) can be the angle formed at an intersection of the second axis A2 and the first axis A1. The angle α can measure between about, but not limited to, 30 degrees to about 50 degrees. In one example, such as shown in FIG. 2A, the angle α can be about 45 degrees.

The plurality of radial fins 126 of the first revision component 120 can include various numbers of individual fins, such as, but not limited to, two, three, four, five, seven, eight or other numbers of fins. In one example, such as shown in FIG. 2A, the plurality of radial fins 126 can include six fins. Each fin of the plurality of radial fins 126 can define various three-dimensional shapes, such as, but not limited to, a rectangular prism, a triangular prism, a trapezoidal prism, or other polyhedrons. Each fin of the plurality of radial fins 126 can define a first radial fin surface 140 and a second radial fin surface 142. The first radial fin surface 140 can extend radially outward from an outer collar surface 144 of the collar 116, such as at various angles including, but not limited to, an orthogonal angle. The second radial fin surface 142 can be an end, or outermost, surface of each fin of the plurality of radial fins 126.

The second radial fin surface 142 can extend outwardly from the first radial fin surface 140 at various angles. For example, the second radial fin surface 142 can extend at, but not limited to, about 10 degrees to about 170 degrees relative to the second radial fin surface 142. In some examples, such as shown in FIG. 2A, the first radial fin surface 140 of each fin of the plurality of radial fins 126 can also extend parallel to, and laterally offset from, the first axis A1. In such an example, the first radial fin surface 140 can define a third axis A3 (FIG. 2A) extending parallel to, and laterally offset from, the first axis A1 when the first revision component 120 is secured to the mounting boss 108. Such an arrangement can enable a surgeon to secure the first revision component 120 to the first component 102 before the modular implant system 100 is implanted into a patient, which can, for example, help the surgeon to perform an arthroplasty procedure with less difficulty and in a reduced amount of time.

In some examples, such as shown in FIG. 1, the plurality of radial fins 126 can form a symmetric arrangement. In a symmetric arrangement, each fin of the plurality of radial fins 126 can be similarly sized and shaped, and can be spaced or otherwise distributed equidistantly, about the outer collar surface 144 relative to other fins of the plurality of radial fins 126. In other examples, such as illustrated in FIGS. 2A-2B, the plurality of radial fins 126 can form an asymmetric arrangement. In an asymmetric arrangement, one or more fins of the plurality of radial fins 126 can be differently sized and shaped, or can be spaced non-equidistantly, about the outer collar surface 144 relative to other fins of the plurality of radial fins 126.

The plurality of radial fins 126 can be based on a statistical bone model representing, for example, an average size and shape of the proximal humerus 130 or a proximal femur, or an average size and shape of an osseous envelope 146 of a plurality of different patients. For example, the position of each individual fin of the plurality of radial fins 126 relative to one another, the number of individual fins the plurality of radial fins 126 includes, or other parameters or characteristics of the first revision component 120, can be configured based on a statistical bone model. A statistical bone model can be generated, for example, from imaging data including, but not limited to, CT, X-ray, or Magnetic Resonance Imaging data. In one example, the Zimmer Biomet ZiBRA Bone Resection Atlas (hereinafter the "ZiBRA Atlas"), which includes imaging data representing aggregated bone morphology from a diverse global population, can be used to create such a statistical bone model.

In some examples, the modular implant system 100 can include a plurality of first revision components, such as, but not limited to, two, three, four, or five uniquely configured first revision components to thereby form a standardized range of different shapes and sizes for the plurality of radial fins 126. This can enable a surgeon to conveniently select the first revision component 120 therefrom, based on one or more physical characteristics of a patient's bone, such as an amount of the metaphysis 134 that can be preserved after a humeral resection, the natural size and shape of a patient's proximal humerus, or natural the size and shape of the osseous envelope 146 (FIG. 2B) of the proximal humerus 130. The osseous envelope 146 can generally be an anatomical void exposed after removal of a previously implanted stemmed prosthetic component, such as a humeral or femoral implant. In a further example, the plurality of radial fins 126 can be based on imaging data collected from an individual patient, such as to help enable the first revision component 120 engage abnormal humeral geometry falling outside a range a plurality of first revision components is sized and shaped to engage. In such an example, it can be appreciated that the first revision component 120 can be configured, and manufactured, specifically for the individual patient.

The first revision component 120 can include a porous surface 150 (FIG. 2A). For example, a portion, or all, of a surface area of the outer collar surface 144, the first radial fin surface 140, or the second radial fin surface 142, can include the porous surface 150. The porous surface 150 can generally be a textured or a patterned three-dimensional structure adapted to help facilitate post-operative bone ingrowth and vascularization. In one example, the porous surface 150 can be realized using Zimmer Biomet OsseoTi^{®} Porous Metal Technology, which uses human CT imaging data in combination with 3D printing technology to build a structure that mimics the architecture of human cancellous bone. In other examples, the porous surface 150 can be realized using Zimmer Biomet Proximal PPS@ Porous Plasma Spray.

The first revision component 120 can be adapted, and selected by a surgeon, for arthroplasties where a greater portion, such as about, but not limited to, any portion of the epiphysis 132, or about 90 percent to about 50 percent of the metaphysis 134, can be preserved or otherwise remains after a humeral resection has been performed. First, for example, the plurality of radial fins 126 can be sized and shaped to engage cancellous bone within the proximal humerus 130 without penetrating cortical bone, such as in a total shoulder arthroplasty or a reverse shoulder arthroplasty, when the first revision component 120 is positioned within the proximal humerus 130. In other examples, the plurality of radial fins 126 can be sized and shaped to fill the osseous envelope 146 by contacting an inner bone surface 148 (FIG. 2B) of cancellous bone defining the osseous envelope 146, such as in a revision shoulder arthroplasty performed to replace a prosthetic humeral implant, when the first revision component 120 is positioned within the osseous envelope 146.

In view of the above, when the first revision component 120 is inserted into the proximal humerus 130, the plurality of radial fins 126 can engage a relatively large amount of surface area within the proximal humerus 130. As such, the plurality of radial fins 126 can allow the modular implant system 100 to effectively transfer stress forces generated during normal joint movement into the epiphysis 132 and the metaphysis 134, to thereby help to prevent post-operative stress-shielding of the proximal humerus 130 and, in turn, help to maintain long-term bone health and strength.

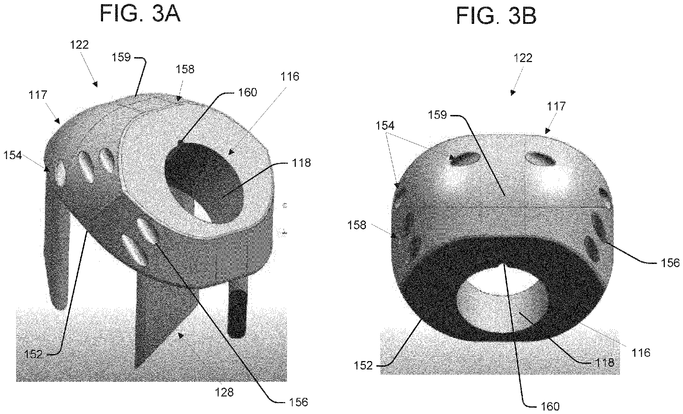

FIG. 3A illustrates an isometric view of an example second revision component 122. FIG. 3B illustrates a rear view of an example second revision component 122. FIGS. 4A and 4B illustrate isometric views of an example modular implant system 100 include a second revision component 122. FIGS. 3A-4B are discussed below concurrently. Also shown in FIG. 4A is a first axis A1, the proximal humerus 130, and the diaphysis 136. Relative to FIG. 2B, the proximal humerus 130 is shown with the epiphysis 132 (FIG. 2B) and the metaphysis 134 (FIG. 2B) resected. The head portion 117 can define the collar 116 of the second revision component 122. The head portion 117 can form various three-dimensional shapes, such as, but not limited to, a semi-hemispherical or a semi-ellipsoidal shape.

The head portion 117 can define a planar surface 152 and a plurality of passages 154. The planar surface 152 can be a flattened bottom surface of the second revision component 122. Each passage of the plurality of passages 154 (FIG. 3B) can be an annular or ellipsoidal aperture, bore, or recess extending through, or into, the head portion 117. The plurality of passages 154 can include various numbers of passages, such as, but not limited to, one, two, three, four, five, six, seven, eight, nine, or ten passages. The plurality of passages 154 can be distributed about the head portion 117 in various positions and orientations. Each passage of the plurality of passages 154 can define a diameter measuring between about, but not limited, 0.5 millimeters to about 3 millimeters, such as to enable a variety of different commercially available sutures to be threaded there through, or a variety of different suture anchors, to be received therein. Such a suture can be, for example, but not limited to, a Maxbraid^{®} or Trulink^{®} suture, or Broadband^{®} Tape, available from Zimmer Biomet; and such a suture anchor can be, for example, but not limited to, a JuggerKnot^{®}, SureLock^{®}, or Quattro X^{®} suture anchor available from Zimmer Biomet.

In some examples, the head portion 117 can define a plurality of angled surfaces 156 (FIG. 3A). The plurality of angled surfaces 156 can generally be a chamfered, tapered, or contoured surface extending between each passage of the plurality of passages 154 and an outer head surface 158 of the head portion 117. The plurality of angled surfaces 156 can thereby help to reduce wear, and extend the life of, a suture located within or extending through one or more passages of the plurality of passages 154. The outer head surface 158 can include a lateral surface 159. The lateral surface 159 can be a lateral, or otherwise a generally outwardly facing, portion of the outer head surface 158 relative to a patient. The lateral surface 159 can be adapted to tension and dictate a wrap angle of a deltoid muscle of a patient's rotator cuff. For example, the lateral surface 159 can be sized and shaped to engage and support a patient's deltoid muscle, such as to apply an amount of initial tension or pre-stretch to the deltoid muscle after the modular implant system 100 has been implanted into a patient. In some examples, a lateral distance, or differential height, between a medial surface 161 of the head portion 117, or an end surface 163 of the mounting boss 108, can be increased, such as to increase the deltoid wrap angle or increase the amount of tension or pre-stretch, or can be decreased, such as to decrease the deltoid wrap angle or decrease the amount of tension or pre-stretch. In some such examples, the lateral surface 159 can further define or form one or more bumps, protrusions, or other three-dimensional shapes adapted to help to contact and engage a deltoid muscle, such as to help increase or decrease a deltoid wrap angle or an amount of deltoid tension or pre-stretch, to thereby further customize the head portion 117 to the needs of an individual patient.

In some examples, the collar 116 of any of the plurality of second components 104 (FIG. 1) can include a slot 160 (FIGS. 3A-3B), and the mounting boss 108 (FIGS. 4A-4B) can include a projection 162 (FIGS. 4A-4B). The slot 160 can be a spline or a groove defined by the first coupling surface 118 (FIGS. 3A-3B). The projection 162 can be a longitudinal protrusion or spline extending radially outward from the outer coupling surface 114 (FIGS. 4A-4B). The slot 160 can be sized and shaped to receive the projection 162 to thereby prevent relative rotation between the outer coupling surface 114 and the first coupling surface 118, when the second revision component 122 is secured to the first component 102 (FIGS. 4A-4B).

The plurality of longitudinal fins 128 can extend longitudinally outward from the planar surface 152 of the head portion 117. The plurality of longitudinal fins 128 can be distributed around the collar 116 and the first coupling surface 118 in an annular or semi-annular arrangement. The plurality of longitudinal fins 128 of the second revision component 122 can include various numbers of individual fins, such as, but not limited to, two, three, five, six, or other numbers of fins. In one example, such as shown in FIGS. 3A, 4A, and 4B, the plurality of longitudinal fins 128 can include four fins. Each fin of the plurality of longitudinal fins 128 can define various of three-dimensional shapes, such as, but not limited to, a rectangular prism, a triangular prism, a trapezoidal prism, or other polyhedrons.

Each fin of the plurality of longitudinal fins 128 can define a first longitudinal fin surface 164 (FIG. 4A) and a second longitudinal fin surface 166 (FIG. 4A). The first longitudinal fin surface 164 can be adapted to extend at various angles relative to the first axis A1 when the second revision component 122 is positioned on the mounting boss 108. In one example, such as shown in FIG. 4A, the first longitudinal fin surface 164 can extend parallel to, and laterally offset from, the first axis A1 when the second revision component 122 is positioned on the mounting boss 108. The second longitudinal fin surface 166 can be an end, or lowermost, surface of each fin of the plurality of longitudinal fins 128. The second longitudinal fin surface 166 can extend at various acute angles such as, but not limited to, between about 10 degrees to about 89 degrees relative to the first longitudinal fin surface 164 to help the plurality of longitudinal fins 128 penetrate cancellous bone of the proximal humerus 130.

In some examples, such as illustrated in FIGS. 3A, 4A, and 4B, the plurality of longitudinal fins 128 can form an asymmetric arrangement. In an asymmetric arrangement, one or more fins of the plurality of longitudinal fins 128 can be differently sized and shaped, or can be spaced non-equidistantly, about the planar surface 152 relative to other fins of the plurality of longitudinal fins 128. In other examples, the plurality of longitudinal fins 128 can form a symmetric arrangement. In a symmetric arrangement, each fin of the plurality of longitudinal fins 128 can be similarly sized and shaped; and can be spaced or otherwise distributed equidistantly about the planar surface 152 relative to other fins of the plurality of longitudinal fins 128. In some examples, the plurality of longitudinal fins 128 can be sized and shaped to circumferentially encompass a portion of the proximal humerus 130, such as to help prevent the proximal humerus 130 from splitting or fracturing.

The head portion 117 and the plurality of longitudinal fins 128 can be based on a statistical bone model representing, for example, an average size and shape of the proximal humerus 130 or a proximal femur of a plurality of different patients. For example, the size and shape of the head portion 117, including the size and shape of the outer head surface 158 and the lateral surface 159, or each individual fin of the plurality of longitudinal fins 128, the orientation or position of each passage of the plurality of passages 154, the number of individual passages of the plurality of passages 154, the position of each individual fin of the plurality of longitudinal fins 128 relative to one another, the number of individual fins the plurality of longitudinal fins 128 includes, or other parameters or characteristics of the second revision component 122, can be configured based on a statistical bone model. A statistical bone model can be generated, for example, from imaging data including, but not limited to, CT, X-ray, or Magnetic Resonance Imaging data. In one example, the ZiBRA Bone Resection Atlas, which includes imaging data representing aggregated bone morphology from a diverse global population, can be used to create such a statistical bone model.

In some examples, the modular implant system 100 can include a plurality of second revision components, such as, but not limited to, two, three, four, or five uniquely configured second revision components to thereby form a standardized range of different shapes and sizes for the head portion 117, including the lateral surface 159, and the plurality of longitudinal fins 128. This can enable a surgeon to conveniently select the second revision component 122 therefrom, based on one or more physical characteristics of a patient's bone, such as an amount of the metaphysis 134 (FIG. 2B) that can be preserved after a humeral or femoral resection, or the natural size and shape of a patient's proximal humerus or proximal femur. For example, the second revision component 122 shown in FIGS. 3A-3B can be configured for an arthroplasty involving a greater amount of bone preservation, and the second revision component 122 shown in FIGS. 4A-4B can be configured by a surgeon in an arthroplasty involving a lesser amount of bone preservation.

Moreover, the second revision component 122 shown in FIGS. 4A can be configured for an arthroplasty involving a smaller humerus or femur; and the second revision component 122 shown in FIG. 4B can be configured for an arthroplasty involving a larger humerus or femur. This can enable a surgeon to conveniently select the second revision component 122 based on amount of the metaphysis 134 that can be preserved after a humeral resection is performed, one or more dimensions of a patient's bone, or other physical characteristics of a patient's bone. Additionally, such a standardized range of different shapes and sizes for the head portion 117 can enable a surgeon to conveniently select the second revision component 122 therefrom based on a desired deltoid wrap angle, or an amount of deltoid tension or pre-stretch, for a patient's deltoid muscle. For example, the second revision component 122 shown in FIG. 4A can be configured for an arthroplasty where a lesser wrap angle, or a lesser amount of deltoid tension or pre-stretch, is desired; and the second revision component 122 shown in FIG. 4B, or a second revision component where the lateral surface 159 defines one or more bumps, protrusions, or other three-dimensional shapes, can be configured for an arthroplasty where a greater wrap angle, or a lesser amount of deltoid tension or pre-stretch is desired. In a further example, the head portion 117 and the plurality of longitudinal fins 128 can be based on imaging data collected from an individual patient, such as to help enable the second revision component 122 to engage abnormal humeral geometry falling outside a range a plurality of second revision components is sized and shaped to engage. In such an example, it can be appreciated that the first revision component 120 can be configured, and manufactured, specifically for the individual patient.

The second revision component 122 can include a porous surface 168 (FIG. 4A). For example, a portion, or all, of a surface area of the planar surface 152, the outer head surface 158, the first longitudinal fin surface 164, or the second longitudinal fin surface 166, can include the porous surface 168. The porous surface 168 can generally be a textured or a patterned three-dimensional structure adapted to help facilitate post-operative bone ingrowth and vascularization. In one example, the porous surface 168 can be realized using Zimmer Biomet OsseoTi^{®} Porous Metal Technology, which uses human CT imaging data in combination with 3D printing technology to build a structure that mimics the architecture of human cancellous bone. In other examples, the porous surface 150 can be realized using Zimmer Biomet Proximal PPS@ Porous Plasma Spray. In an additional example, the plurality of longitudinal fins 128 can be made using an additive manufacturing technique, in conjunction with Zimmer Biomet OsseoTi^{®} Porous Metal Technology, to enable each fin of the plurality of longitudinal fins 128 to be, or otherwise form, a substantially or entirely porous structure that mimics the architecture of human cancellous bone.

The second revision component 122 can be adapted for use in arthroplasties where a lesser portion, such as between, but not limited to, about 50 percent to about 0 percent of the metaphysis 134 (FIG. 4A), or a metaphysis of a femur, can be preserved or otherwise remains after a resection has been performed. First, the head portion 117 can be adapted to replace various bony landmarks of the humerus or femur to help restore natural humeral or femoral geometry. For example, the head portion 117 can be sized and shaped to maintain a natural humeral height of the proximal humerus 130 (e.g., the height of a greater tuberosity relative to a natural height of a humeral head), such as created by filling an anatomical void within a patient created through resection of the proximal humerus 130, such as including resection of a portion or all of a greater or lesser tuberosity thererof. Additionally, the Moreover, the plurality of passages 154 can be positioned, and sized, to enable a surgeon to secure the supraspinatus, subscapularis, or other tendons of the rotator cuff to the second revision component 122 in natural or anatomically correct locations. This can allow the head portion 117 to restore natural shoulder joint biomechanics and reduce long-term rotator cuff inflammation or irritation.

Second, the second revision component 122 can be adapted to resist rotational and lever-out forces to provide enhanced stability and improve bone post-operative ingrowth. For example, the planar surface 152 can be sized and shaped to rest on the outer bone surface 138 of the proximal humerus 130; and the plurality of longitudinal fins 128 can be sized and shaped to extend into cancellous bone of the metaphysis 134 or the diaphysis 136 to secure the second revision component 122 to the proximal humerus 130. In view of the above, by improving contact and engagement with a patient's bone, the second revision component 122 can help prevent post-operative stress-shielding to thereby reduce the possibility of diminished or otherwise reduced long-term bone health and strength.

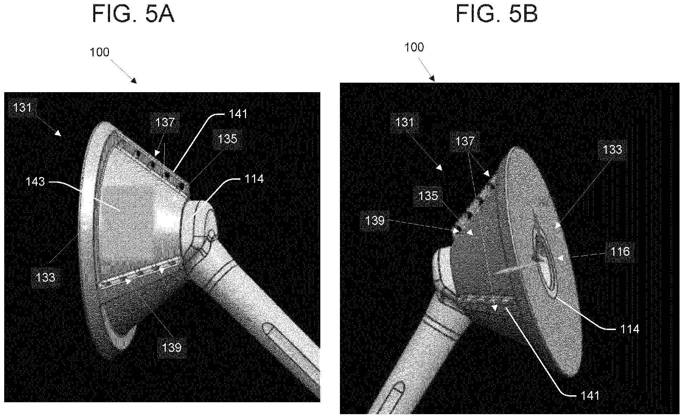

FIGS. 5A-5B illustrate side views of an example modular implant system 100 including a third revision component 131. In some examples, the plurality of second components 104 (FIG. 1) of the modular implant system 100 can include a third revision component 131. The third revision component 131 can include an inner portion 133 and an outer portion 135. The inner portion 133 and the outer portion 135 can collectively define the collar 116 (FIG. 5B) of the third revision component 131. The inner portion 133 can form various three-dimensional shapes, such as, but not limited to, a frustoconcial shape. The outer portion 135 can form various three-dimensional shapes, such as, but not limited to, an annular shape. The inner portion 133 can include a plurality of projections 137 extending outward therefrom. Each projection of the plurality of projections 137 can define various shapes, such as, but not limited to, a rectangular three-dimensional body. Each projection of the plurality of projections 137 can define a plurality of transverse passages 139 extending there through, such as, but not limited to, in a linear arrangement. Each passage of the plurality of transverse passages 139 can be an annular or ellipsoidal aperture, bore, or recess extending through, or into, a projection of the plurality of projections 137.

The plurality of transverse passages 139 of each projection of the plurality of projections can include various numbers of individual passages, such as, but not limited to, one, two, three, or four passages. Each passage of the plurality of transverse passages 139 can also extend at various angles or orientations, relative to one another or to an outer surface 141 of each projection of the plurality of projections 137. Each passage of the plurality of transverse passages 139 can define a diameter measuring between about, but not limited, 0.5 millimeters to about 3 millimeters, such as to enable a variety of different commercially available sutures to be threaded there through, or a variety of different suture anchors to be received therein. Such a suture can be, for example, but not limited to, a Maxbraid^{®} or Trulink^{®} suture, or Broadband^{®} Tape, available from Zimmer Biomet; and such a suture anchor can be, for example, but not limited to, a JuggerKnot^{®}, SureLock^{®}, or Quattro X^{®} suture anchor available from Zimmer Biomet.

The inner portion 133 and the outer portion 135 can be based on a statistical bone model representing, for example, an average size and shape of the proximal humerus 130 or a proximal femur of a plurality of different patients. For example, the size and shape of the inner portion 133 or the outer portion 135, the plurality of projections 137, the orientation or position of each passage of the plurality of transverse passages 139, or the number of individual passages of the plurality of transverse passage 139 includes, or other parameters or characteristics of the third revision component 131, can be configured based on a statistical bone model. A statistical bone model can be generated, for example, from imaging data including, but not limited to, CT, X-ray, or Magnetic Resonance Imaging data. In one example, the ZiBRA Bone Resection Atlas, which includes imaging data representing aggregated bone morphology from a diverse global population, can be used to create such a statistical bone model.

In some examples, the modular implant system 100 can include a plurality of third revision components, such as, but not limited to, two, three, four, or five uniquely configured third revision components to thereby form a standardized range of different shapes and sizes for the inner portion 133 and the outer portion 135. This can enable a surgeon to conveniently select the third revision component 131 therefrom, based on one or more physical characteristics of a patient's bone, such as an amount of the metaphysis 134 (FIG. 2B) that can be preserved after a humeral or femoral resection, or the natural size and shape of a patient's proximal humerus or proximal femur.

The third revision component 131 can include a porous surface 143 (FIG. 5A). For example, a portion, or all, of a surface area of the inner portion 133 or the outer portion 135 can include the porous surface 143. The porous surface 143 can generally be a textured or a patterned three-dimensional structure adapted to help facilitate post-operative bone ingrowth and vascularization. In one example, the porous surface 143 can be realized using Zimmer Biomet OsseoTi^{®} Porous Metal Technology, which uses human CT imaging data in combination with 3D printing technology to build a structure that mimics the architecture of human cancellous bone. In other examples, the porous surface 143 can be realized using Zimmer Biomet Proximal PPS@ Porous Plasma Spray.

The third revision component 131 can be adapted for use in arthroplasties where a lesser portion, such as between, but not limited to, about 50 percent to about 0 percent of the metaphysis 134 (FIG. 4A), or a metaphysis of a femur, can be preserved or otherwise remains after a resection has been performed. In some examples, the third revision component 131 may be used in arthroplasties where cancellous bone of the metaphysis 134 or the diaphysis 136 (FIG. 4A) is significantly deteriorated or degraded, such to a point where use of the first revision component 120 (FIGS. 2A-2B) or the second revision component (FIGS. 3A-4B) may involve the use of bone cement, bone grafts, or other additional fixation techniques to secure the first revision component 120 or the second revision component 122 to, or within, the proximal humerus 130 (FIGS. 2B and 4A). First, the inner portion 133 and the outer portion 135 and the can be adapted to replace various bony landmarks of the humerus or femur to help restore natural humeral or femoral geometry. For example, the outer portion 135 can be sized and shaped to maintain a natural humeral height of the proximal humerus 130 (e.g., the height of a greater tuberosity relative to a natural height of a humeral head), such as created by filling an anatomical void within a patient created through resection of the proximal humerus 130), such as including resection of a portion or all of a greater or lesser tuberosity thererof. Moreover, the plurality of transverse passages 139 of the inner portion 133 can be positioned, and sized, to enable a surgeon to secure the supraspinatus, subscapularis, or other tendons of the rotator cuff to the third revision component 131 in natural or anatomically correct locations. This can allow the third revision component 131 to restore natural shoulder joint biomechanics and reduce long-term rotator cuff inflammation or irritation.

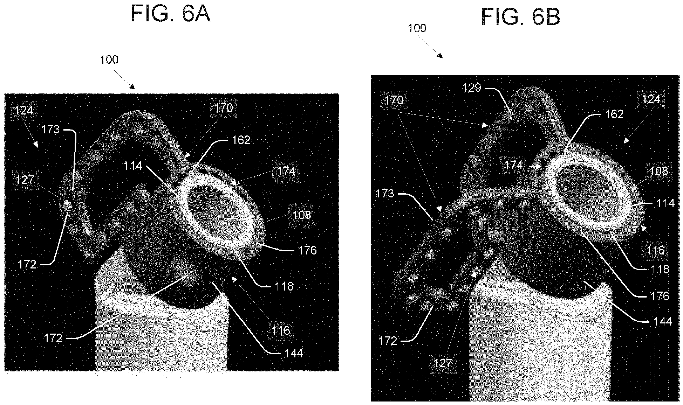

FIGS. 6A-7B illustrate isometric views of an example first fracture component 124. FIGS. 6A-7B are discussed below concurrently. The first fracture component 124 can include at least one fin 170. In one example, the at least one fin 170 can include a single fin, such as shown in FIG. 5A. In another example, the at least one fin 170 can include two individual fins, such as shown in FIG. 6B. The at least one fin 170 can extend radially outward from the outer collar surface 144 of the collar 116. The at least one fin 170 can be spaced circumferentially, or radially, apart relative to one another around the collar 116 by about, but not limited to, 45 degrees to about 60 degrees. In other examples, the at least one fin 170 include can three, four, or other numbers of individual fins, such as located equidistantly or non-equidistantly relative to one another around the outer collar surface 144.

Each fin of the at least one fin 170 can define a plurality of first apertures 127. Each aperture of the plurality of first apertures 127 can be spaced laterally apart, relative to one another, along each fin of the at least one fin 170. Each aperture of the plurality of apertures can extend transversely through each fin of the at least one fin 170. The plurality of first apertures 127 can include various numbers of individual apertures, such as between, but not limited to, about five individual apertures to about fifteen individual apertures. Each aperture of the plurality of first apertures 127 can define a diameter measuring between about, but not limited to, 0.8 millimeters to about 3 millimeters, such as to enable a wide variety of commercially available sutures to be threaded through each fin of the at least one fin 170.

In some examples, the at least one fin 170 can also include a plurality of angled surfaces 172. Each angled surface of the plurality of angled surfaces 172 can generally be a chamfered, tapered, or contoured surface extending between a fin surface 173 and each aperture of the plurality of first apertures 127. The fin surface 173 can generally represent a medial or lateral, or first or second, planar side surface of each fin of the at least one fin 170. The plurality of angled surfaces 172 can thereby help to reduce wear, and extend the life of, of a suture located within an aperture of the plurality of first apertures 127.

In some examples, the first fracture component 124 can include a plurality of slots 174. The plurality of slots 174 can be a plurality of female splines or grooves defined by the first coupling surface 118. The projection 162 of the mounting boss 108, and the plurality of slots 174, can extend parallel to and laterally offset from the second axis A2 (FIG. 2A) when the first fracture component 124 is secured to the mounting boss 108. The plurality of slots 174 can form a radial or annular arrangement about the first coupling surface 118, such as extending completely, or partially, around a circumference of the first coupling surface 118. Each slot of the plurality of slots 174 can be sized and shaped to receive the projection 162 to thereby prevent relative rotation between the outer coupling surface 114 and the first coupling surface 118.

In some examples, the first fracture component 124 can be based on a statistical bone model representing, for example, a calculated average size and shape of one or more displaced fractures (e.g., bone fragments), such as one, two, or three displaced fractures, or a calculated average size and shape of a proximal humerus or proximal femur, of a variety of different patients. For example, the size and shape of the at least one fin 170, the number of individual fins the at least one fin 170 includes, the position or orientation of each aperture of the plurality of first apertures 127 within the at least one fin 170, or the number of individual apertures of the plurality of first apertures 127, or other parameters or characteristics of the first fracture component 124, can be configured based on a statistical bone model. A statistical bone model can be generated, for example, from imaging data including, but not limited to, CT, X-ray, or Magnetic Resonance Imaging data. In one example, the Zimmer Biomet ZiBRA Bone Resection Atlas (hereinafter the "ZiBRA Atlas"), which includes imaging data representing aggregated bone morphology from a diverse global population, can be used to create such a statistical bone model.

In some examples, the modular implant system 100 can include a plurality of first fracture components, such as, but not limited to, two, three, four, or five uniquely configured first fracture components to thereby form a standardized range of different shapes and sizes for the at least one fin 170. This can enable a surgeon to conveniently select the first fracture component 124 therefrom based on one or more physical characteristics of a patient's bone, such as the size and shape, and number, of displaced humeral fractures. For example, the first fracture component 124 shown in FIG. 5A can be configured for an arthroplasty involving a three-part humeral fracture; and the first fracture component 124 shown in FIGS. 5A-5B, or 6A-6B, can be configured for an arthroplasty involving a four-part humeral fracture. In various examples, the number of individual fins the at least one fin 170 includes, the size or shape of each fin of the at least one fin 170, the circumferential or radial spacing between each fin of the at least one fin 170, or the number of apertures of the plurality of first apertures 127 each planar fin defines, can be different between each first fracture component 124 of such a plurality of first fracture components, such as to further help a surgeon to find a best fit for the needs of an individual patient.

In some examples, the first fracture component 124 can include a porous surface 176 (FIG. 5A). For example, a portion, or all, of a surface area of the outer collar surface 144, the fin surface 173, or other surfaces of the first fracture component 124 can include the porous surface 176. The porous surface 176 can generally be a textured or a patterned three-dimensional structure adapted to help facilitate post-operative bone ingrowth and vascularization. In one example, the porous surface 176 can be realized using Zimmer Biomet OsseoTi^{®} Porous Metal Technology, which uses human CT imaging data in combination with 3D printing technology to build a structure that mimics the architecture of human cancellous bone. In other examples, the porous surface 176 can be realized using Zimmer Biomet Proximal PPS@ Porous Plasma Spray.

The first fracture component 124 can be adapted for use in arthroplasties where one or more displaced bone fragments, such as of a proximal humerus or a proximal femur, can be preserved through fixation to the modular implant system 100. For example, the at least one fin 170 can support one or more displaced humeral bone fragments in anatomically accurate positions within a patient to restore natural humeral or femoral geometry. In one example, in an arthroplasty involving a three-part or a four-part humeral fracture, and a first bone fragment including a greater tuberosity and a second fragment including a lesser tuberosity, a surgeon can reconstruct natural humeral geometry, such as by threading one or more sutures through the plurality of first apertures 127, and wrapping the one or more sutures circumferentially around the first bone fragment, the second bone fragment, and the outer collar surface 144 to secure the first bone fragment and the second bone fragment to the first fracture component 124. The surgeon can then reattach the supraspinatus of rotator cuff to the first bone fragment and the subscapularis of the rotator cuff to the second bone fragment to restore joint functionality.

Additionally, the plurality of slots 174 and the projection 162 can enable a surgeon to selectively position the first fracture component 124 on the mounting boss 108 in an orientation best suited for the anatomical needs of an individual patient. For example, a surgeon can select one slot of the plurality of slots 174 which best locates the at least one fin 170, such as aligning the at least one fin 170 with one or more natural fracture lines or surfaces of one or more displaced bone fragments, or best positions the at least one fin 170 to accommodate the sizes and shapes of displaced bone fragments. In a further example, the at least one fin 170 can be made from a malleable or bendable material to enable surgeon to selectively shape, such as by bending, the at least one fin 170, to further customize the at least one fin 170 to one or more natural fracture lines or surfaces of one or more displaced bone fragments, or best positions the at least one fin 170 to accommodate the sizes and shapes of displaced bone fragments.

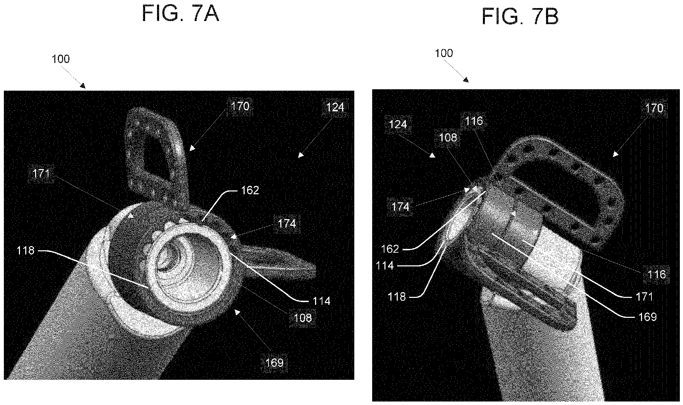

In some examples, such as shown in FIGS. 7A-7B, the first fracture component 124 can be realized in the form of two individual and separate components. For example, the collar 116 can include, or otherwise be formed by, a first collar 169 and a second collar 171. Each of the first collar 169 and the second collar 171 can include one or more fins of the at least one fin 170. least one fin of the at least one fin 170. This can help enable a user to selectively position the first fracture component 124 on the mounting boss 108 in an orientation best suited for the anatomical needs of an individual patient. For example, a surgeon can select a slot of the plurality of slots 174 for each of the first collar 169 and the second collar 171 which best locates each of the at least one fin 170 for the needs of an individual patient, such as by aligning the at least one fin 170 with one or more natural fracture lines or surfaces of one or more displaced bone fragments, or best positions the at least one fin 170 according to a fracture pattern or the sizes and shapes of displaced bone fragments.

Moreover, as the first collar 169 can be selected independently of the second collar 171, a surgeon can select, for example, a first collar having a differently sized and shaped at least one fin 170 relative to an at least one fin 170 of the second collar 171. This can further increase the number of customization options available to a surgeon in fracture correction arthroplasties. In view of all the above, the first fracture component 124 can provide a surgeon with wide variety of customization options to help accurately reconstruct natural humeral geometry, and thereby help restore joint functionality, in fracture correction arthroplasties.

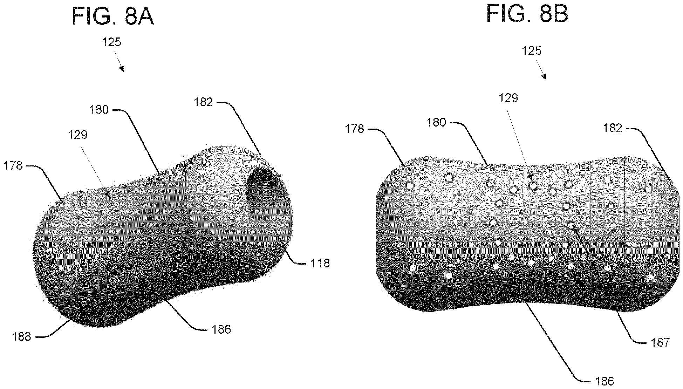

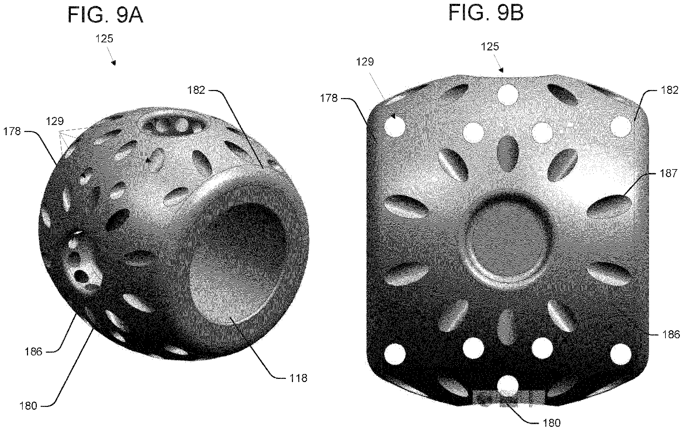

FIG. 8A illustrates an isometric view of an example second fracture component 125. FIG. 8B illustrates a side view of an example second fracture component 125. FIGS. 9A-9B illustrate isometric views of an example second fracture component 125. FIGS. 8A- 9B are discussed below concurrently. The second fracture component 125 can include a first end portion 178, a central portion 180, and a second end portion 182. The first end portion 178 and the second end portion 182 can form various three-dimensional shapes, such as, but not limited to, a semi-hemispherical or an ellipsoidal shape. The central portion 180 can form various three-dimensional shapes, such as, but not limited to, a concave or convex cylindrical or an ellipsoidal shape. In some examples, such as shown in FIGS. 8A-8B, the first end portion 178 and the second end portion 182 can define a greater diameter than a diameter defined by the central portion 180. In other examples, such as shown in FIGS. 9A-9B, the first end portion 178 and the second end portion 182 can define a lesser diameter than a diameter defined by the central portion 180.

The second fracture component 125 can define an outer surface 186. The outer surface 186 can be an outermost surface of the second fracture component 125 and the first coupling surface 118 (FIGS. 8A & 9A) can be an innermost surface of the second fracture component 125. Each aperture of the plurality of second apertures 129 can generally be an annular or ellipsoidal bore or recess extending through, or into, the second fracture component 125. In some examples, one or more apertures of the plurality of second apertures 129 can extend radially through the second fracture component 125 between the outer surface 186 and the first coupling surface 118, or can extend into or through the second fracture component 125 vertically, or laterally offset from, the outer surface 186 or the first coupling surface 118. The plurality of second apertures 129 can include various numbers of individual second apertures, such as, but not limited to, between about ten apertures and about thirty second apertures.

Each aperture of the plurality of second apertures 129 can define a diameter measuring between about, but not limited to, 0.8 millimeters to about 3 millimeters, such as to enable a variety of different commercially available sutures to be threaded there through, or a suture anchor to be received therein. Such a suture can be, for example, but not limited to, a Maxbraid^{®} or Trulink^{®} suture, or Broadband^{®} Tape, available from Zimmer Biomet; and such a suture anchor can be, for example, but not limited to, a JuggerKnot^{®}, SureLock^{®}, or Quattro X^{®} suture anchor available from Zimmer Biomet. In some examples, each passage of the plurality of second apertures 129 can define at least one angled surface 187 (FIGS. 8B & 9B). The at least one angled surface 187 can generally be chamfered, tapered, or contoured end surface of each aperture of the plurality of second apertures 129, such as defined in the outer surface 186 or the first coupling surface 118. The at least one angled surface 187 can help to reduce wear on a suture extending one or more apertures of the plurality of second apertures 129. The plurality of second apertures 129 can be distributed about the second fracture component 125 in a variety of different positions and orientations. For example, the plurality of second apertures 129 can form one or more radial arrangements, such as located entirely within the central portion 180, as shown in FIG. 8A or FIGS. 9A-9B, or partially or otherwise across the central portion 180 and the first end portion 178, or the second end portion 182, such as shown in FIGS. 9A-9B.

In other examples, some apertures of the plurality of second apertures 129 can form a radial arrangement within the central portion 180, and other apertures of the plurality of second apertures 129 can be distributed evenly or unevenly throughout the first end portion 178 or the second end portion 182, such as shown in FIGS. 8B, 9A, and 9B. In still further examples, the plurality of second apertures 129 can form other arrangements, such a substantially liner arrangement across the first end portion 178, the central portion 180, and the second end portion 182, or a relatively continuous or equidistant distribution pattern throughout the first end portion 178, the central portion 180, and the second end portion 182.

In some examples, the second fracture component 125 can be based on a statistical bone model representing, for example, a calculated average size and shape of one or more displaced fractures (e.g., bone fragments), such as one, two, or three displaced fractures, or a calculated average size and shape of a proximal humerus or proximal femur, of a variety of different patients. For example, the size and shape of the first end portion 178, the central portion 180, the second end portion 182, the position of each aperture of the plurality of second apertures 129, or the number of individual apertures of the plurality of second apertures 129, or other parameters or characteristics of the second fracture component 125, can be configured based on a statistical bone model. A statistical bone model can be generated, for example, from imaging data including, but not limited to, CT, X-ray, or Magnetic Resonance Imaging data. In one example, the Zimmer Biomet ZiBRA Bone Resection Atlas (hereinafter the "ZiBRA Atlas"), which includes imaging data representing aggregated bone morphology from a diverse global population, can be used to create such a statistical bone model.

In some examples, the modular implant system 100 (FIG. 1) can include a plurality of second fracture components, such as, but not limited to, two, three, four, or five uniquely configured second fracture components to thereby form a standardized range of different shapes and sizes for the first end portion 178, the central portion 180, the second end portion 182, and standardized range of different arrangements for the plurality of second apertures 129. This can enable a surgeon to conveniently select the second fracture component 125 therefrom, based on one or more physical characteristics of a patient's bone, such as, but not limited to, the size, shape, and number of humeral or femoral bone fragments of the patient. In a further example, the first end portion 178, the central portion 180, and the second end portion 182 can be based on imaging data collected from an individual patient, such as to help enable the second fracture component 125 accommodate displaced bone fracture falling outside a range a plurality of second fracture components is sized and shaped to accommodate. In such an example, it can be appreciated that the second fracture component 125 can be configured, and manufactured, specifically for the individual patient.

The second fracture component 125 can include a porous surface 188 (FIG. 8A). For example, a portion, or all, of a surface area of the outer surface 186 of the first end portion 178, the central portion 180, or the second end portion 182 can include the porous surface 188. The porous surface 188 can generally be a textured or a patterned three-dimensional structure adapted to help facilitate post-operative bone ingrowth and vascularization. In one example, the porous surface 188 can be realized using Zimmer Biomet OsseoTi^{®} Porous Metal Technology, which uses human CT imaging data in combination with 3D printing technology to build a structure that mimics the architecture of human cancellous bone. In other examples, the porous surface 188 can be realized using Zimmer Biomet Proximal PPS@ Porous Plasma Spray.

The second fracture component 125 can be adapted for use in arthroplasties where one or more displaced bone fragments, such as of a proximal humerus or a proximal femur, can be preserved through fixation to the modular implant system 100 (FIG. 1). For example, the first end portion 178 and the second end portion 182 can be sized and shaped relative to the central portion 180 to prevent displaced bone fragments from sliding along the outer surface 186, such as to help retain bone fragments therebetween, and support one or more displaced humeral bone fragments in anatomically accurate positions within a patient to restore natural humeral or femoral geometry. Moreover, the plurality of second apertures 129 can be adapted to allow a surgeon to utilize single or double row of sutures to affix one or more displaced bone fragments directly to the outer surface 186 of the second fracture component 125. For example, the plurality of second apertures 129 can be located and oriented to enable a surgeon to axially align one or more bores drilled through a displaced bone fragment with one or more apertures of the plurality of second apertures 129, such as in a variety of different fracture correction arthroplasties involving displaced bone fragments of different sizes and shapes.

This can enable the surgeon to deploy a first suture anchor containing a first end of a suture into an aperture of the plurality of second apertures, pass the suture through a bore of a displaced bone fragment axially aligned with the aperture, and affix a second end of the suture to the displaced bone fragment, such as by using a second suture anchor deployed within the displaced bone fragment. The surgeon can then reattach the supraspinatus of the rotator cuff to the first bone fragment and the subscapularis of the rotator cuff to the second bone fragment to restore joint functionality. In view of the above, the second fracture component 125 can provide a surgeon with wide variety of customization options to help accurately reconstruct natural humeral geometry, and thereby help restore joint functionality, in fracture correction arthroplasties.

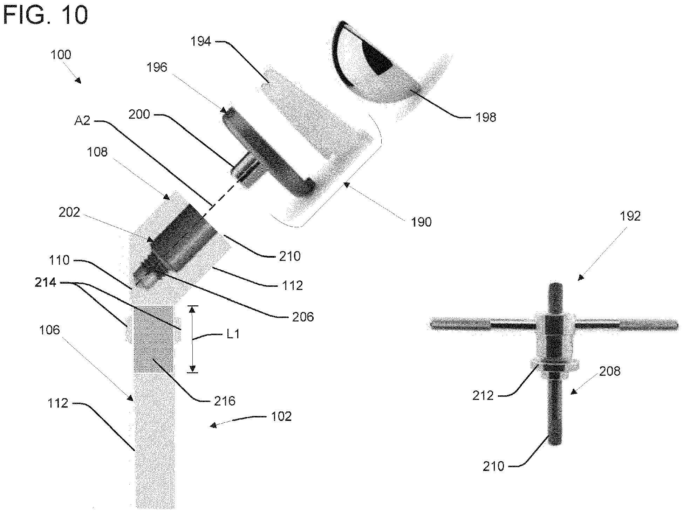

FIG. 10 illustrates a side view of an example modular implant system 100. The modular implant system 100 can include a third component 190 and an assembly tool 192. In FIG. 10, the first component 102 is shown in cross-section. The third component 190 can generally be a system or device adapted to replace a patient's natural humeral head. For example, in a reverse shoulder arthroplasty, the third component 190 can be, but is not limited to, a Zimmer Biomet Comprehensive^{®} or Identity^{®} humeral tray. In such an example, the third component 190 can include a cup 194 and a tray 196. The cup 194 can be a recessed or concave bearing sized and shaped to allow a glenosphere 198 to articulate therein or thereagainst. The tray 196 can be a component adapted to receive a portion of the cup 194. The tray 196 can define a second coupling surface 200; and the mounting boss 108 can define an inner coupling surface 202. The inner coupling surface 202 can be an inner-most surface of the mounting boss 108.

The inner coupling surface 202 can be adapted to contact and engage the second coupling surface 200 to secure the first component 102 to the third component 190. For example, the second coupling surface 200 can define a male taper and the inner coupling surface 202 can define a female taper, such as to form a Morse taper. In another examples, the second coupling surface 200 can be sized and shaped to engage the inner coupling surface 202 via a press fit. In some examples, such as in a total arthroplasty procedure, the third component 190 can, such as alternatively to including the cup 194 and the tray 196 as shown in FIG. 10, be a device or system defining a prosthetic humeral head for articulating within a glenoid fossa or prosthetic glenoid cup. In such an example, the third component 190 can also include the second coupling surface 200 to enable the third component 190 to engage the inner coupling surface 202 and thereby secure the third component 190 to the first component 102. In view of the above, the uniform nature of the surface engagement between the inner coupling surface 202 and the second coupling surface 200 can enable the modular implant system 100 to be configured by a surgeon for both total and reverse arthroplasties.

The mounting boss 108 can define a first plurality of threads 206. The assembly tool 192 can generally be a device adapted to help facilitate controlled coupling of any second component of the plurality of second components 104 (FIG. 1) to the first component 102. In one example, the assembly tool 192 can be a T-handle, such as shown in FIG. 10. In other examples, the assembly tool 192 can be a bit adapted to be engaged by or received within with drill, rachet, or various other powered or non-powered tools. The assembly tool 192 can include a shaft portion 208. The shaft portion 208 can generally be an elongated portion of the assembly tool 192 defining a second plurality of threads 210. The first plurality of threads 206 can be adapted to threadedly engage the second plurality of threads 210 during assembly of the modular implant system 100. For example, when the outer coupling surface 114 of the first component 102 is concentrically aligned with the first coupling surface 118 of a second component of the plurality of second components 104, the shaft portion 208 can be passed through the second component, such as by moving the shaft portion along the second axis A2 (FIG. 2A) towards the mounting boss 108 until the second plurality of threads 210 is at least partially received within the first plurality of threads 206. The shaft portion 208 can then be rotated, such as in a clockwise direction, to cause the second plurality of threads 210 to rotate relative to the first plurality of threads 206 to, in turn, causing the assembly tool 192 to be draw toward the first component 102 along the second axis A2.

The assembly tool 192 can also include a flange 212. The flange 212 can be a body extending radially outward from the shaft portion 208. The flange 212 can be sized and shaped to contact and engage one or more surfaces of any second component of the plurality of second components 104 during assembly of the modular implant system 100. For example, when the second plurality of threads 210 is at least partially received within the first plurality of threads 206, the flange 212 can be in contact with a second component of the plurality of second components 104. Accordingly, when the assembly tool 192 is drawn toward the first component 102 during rotation of the second plurality of threads 210 relative to the first plurality of threads 206, the flange 212 apply an axial force to a second component of the plurality of second components 104 to drive the first coupling surface 118 along the outer coupling surface 114 in a controlled manner.

In some examples, the first component 102 can include one or more fins 214. The one or more fins 214 can extend radially outward from the stem 106, such as from the proximal portion 110 or the distal portion 112. The one or more fins 214 can include various numbers of individual fins, such as, but not limited to, two, three, four, five, seven, eight or other numbers of fins. In one example, such as shown in FIG. 10, the one or more fins 214 can include two fins. Each fin of the one or more fins 214 can define various three-dimensional shapes, such as, but not limited to, a rectangular prism, a triangular prism, a trapezoidal prism, or other polyhedrons. In some examples, such as shown in FIG. 10, the one or more fins 214 can form a symmetric arrangement. In a symmetric arrangement, each fin of the one or more fins 214 can be similarly sized and shaped, and can be spaced or otherwise distributed equidistantly, about the stem 106 relative to other fins of the plurality of radial fins 126.

In other examples, the one or more fins 214 can form an asymmetric arrangement. In an asymmetric arrangement, one or more fins of the one or more fins 214 can be differently sized and shaped, or can be spaced non-equidistantly, about the stem 106 relative to other fins of the one or more fins 214. The one or more fins 214 can be based on a statistical bone model representing, for example, an average size and shape of a metaphysis or diaphysis of a humerus or femur of a plurality of different patients. For example, the position of each individual fin of the one or more fins 214 relative to one another, the number of individual fins the one or more fins 214 includes, other parameters or characteristics of the one or more fins 214, or other parameters or characteristics of the first component 102, such as the size and shape of the stem 106 to help enable the stem 106 match a natural taper defined in a humeral or femoral medullary canal, can be configured based on a statistical bone model. A statistical bone model can be generated, for example, from imaging data including, but not limited to, CT, X-ray, or Magnetic Resonance Imaging data. In one example, the Zimmer Biomet ZiBRA Bone Resection Atlas (hereinafter the "ZiBRA Atlas"), which includes imaging data representing aggregated bone morphology from a diverse global population, can be used to create such a statistical bone model.

The one or more fins 214 can be adapted for arthroplasties where a lesser portion, or none, of a metaphysis of a humerus or femur can be preserved or will remain after a humeral resection has been performed. For example, the plurality of radial fins 126 can be sized and shaped to engage cancellous bone within a humerus or femur, such as without penetrating cortical bone when the stem 106 is inserted into a medullary canal thereof, to help engage surface area within a diaphysis of a humerus or femur. This can help the modular implant system 100 transfer stress forces generated during joint movement into bone, to thereby help prevent post-operative stress-shielding and maintain long-term bone health and strength, where a lesser portion, or none, of a metaphysis of a humerus or femur can be preserved or will remain after a humeral resection has been performed.

The stem 106 can include a porous surface 216. For example, a portion, or all, of a surface area of the stem 106 can include the porous surface 216. The porous surface 216 can extend circumferentially around an outer diameter of the stem 106. The porous surface 216 can define a longitudinal length L1 relative to the first axis A1 (FIG. 2A), such as measuring between about, but not limited to, 2 inches to about 10 inches. In one example, the porous surface 216 can extend between the proximal portion 110 and the distal portion 112 of the stem 106. In another example, the porous surface 216 can extend along the proximal portion 110. The plurality of second components 104, including any of the first revision component 120 (FIG. 1), the second revision component 122 (FIG. 1), the first fracture component 124 (FIG. 1), or the second fracture component 125 (FIG. 1), can be made from a various materials, such as, but not limited to, ceramics, metals such as stainless steel, titanium, or other metal alloys, such as including cobalt or chromium, through three-dimensional printing, metallic molding, machining, additive manufacturing, or other manufacturing techniques.

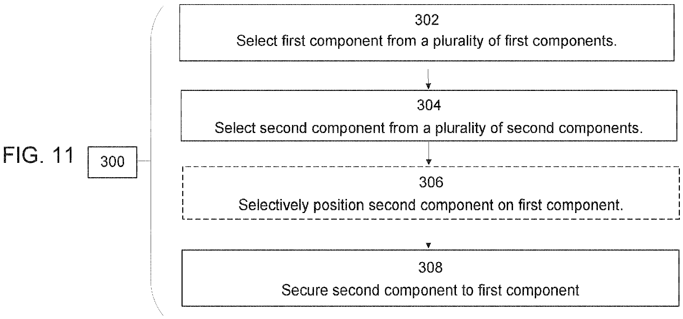

FIG. 11 illustrates a method 300 of adapting a modular implant system to a patient. The steps or operations of the method 300 are illustrated in a particular order for convenience and clarity; many of the discussed operations can be performed by multiple different actors, devices, or systems. It is understood that subsets of the operations discussed in the method 300 can be attributable to a single actor, device, or system and can be considered a separate standalone process or method.

The method 300 can optionally begin with operation 302. The operation 302 can include selecting a first component from a plurality of first components, the first component adapted to be anchored within a medullary canal of a bone of the patient. For example, a surgeon can select the first component from the plurality of first components based on the natural size and shape of the patient's proximal humerus or proximal femur, such as including a length or diameter of a medullary canal thereof. In one such example, a relatively short, or shorter, first component can be selected by a surgeon for a total shoulder arthroplasty or for a reverse shoulder arthroplasty; and a relatively long, or longer, first component can be selected by a surgeon for a revision arthroplasty, such as performed to replace an existing stemmed humeral or femoral implant, or for a fracture correction shoulder arthroplasty, such as performed to reconstruct fractured natural bony landmarks.

In some examples, the operation 302 can include selecting the first component based on a longitudinal length of a porous surface of the first component. For example, a surgeon can select a first component including a porous surface extending along a proximal portion thereof, such as for an arthroplasty where a greater portion of a metaphysis of a proximal humerus or femur can be preserved or will remain after a resection is performed. Alternatively, a surgeon can select a first component including a porous surface extending along or between a proximal portion and a distal portion thereof, such as for an arthroplasty where a lesser portion, or none, of a metaphysis of a proximal humerus or femur that can be preserved or will remain after a resection is performed. This can help to improve post-operative ingrowth and long-term bone strength, to help compensate for a lack of surface engagement between cancellous bone of a metaphysis and the modular implant system.

The method 300 can include operation 304. The operation 304 can include selecting a second component from a plurality of second components based on a physical characteristic of the bone, the second component adapted to engage at least one of a metaphysis or a diaphysis of the bone of the patient. For example, a surgeon can select the second component from the plurality of second components, such as by selecting one of a first revision component, a second revision component, a first fracture component, or a second fracture component, based on the natural size and shape of a patient's proximal humerus or femur, an amount of bone from the metaphysis or diaphysis that can be preserved or will remain after a resection is performed, or the size, shape, and number of humeral or femoral bone fragments.

The method 300 can optionally include operation 306. The operation 306 can include selectively positioning the second component on the first component. For example, a surgeon can first concentrically align an outer coupling surface of the first component with a first coupling surface of the second component. The surgeon can then position the second component about the first component in an orientation best suited for the anatomical needs of an individual patient by inserting a projection of the first component into one slot of a plurality of slots defined in the second component which best locates at least one fin of the second component with respect to one or more natural fracture lines or surfaces of one or bone displaced bone fragments, or otherwise best positions the at least one fin to accommodate the size and shape of one or more displaced bone fragments.

The method 300 can include operation 308. The operation 308 can include securing the second component to the first component. For example, a surgeon can first concentrically align an outer coupling surface of the first component with a first coupling surface of the second component. The surgeon can then apply an axial force to the second component to cause the second component to slide along a mounting boss of the first component toward a stem of the first component. As the second component slides along the mounting boss, the first coupling surface of the second component can slideably engage the outer coupling surface of the mounting boss to secure the second component to the first component.

In some examples, the operation 308 can include inserting an assembly tool through the second component to threadedly engage a first plurality of threads defined within a mounting boss with a second plurality of threads defined by the assembly tool. For example, once the outer coupling surface of the first component is concentrically aligned with the first coupling surface of the second component, a surgeon can insert a shaft portion of the assembly tool through the second component by moving the shaft portion toward the first component, such as along a second axis defined by the mounting boss, until the second plurality of threads of the assembly tool contact and engage the first plurality of threads defined by an inner coupling surface of the mounting boss.

In an alternative example, thermal expansion and contraction can be used to secure the first component to the second component. For example, a surgeon or user can freeze or otherwise cool the first component to cause the first component to contract and heat the second component to cause the second component to expand. Subsequently, a surgeon can concentrically align the outer coupling surface of the first component with the first coupling surface of the second component, and move the second component toward the first component until all, or a portion, of a surface area of the outer coupling surface is contact with all, or a portion, of a surface area of the first coupling surface.

In some examples, the operation 308 can include rotating the assembly tool to cause a first coupling surface defined by the collar to slidably engage an outer coupling surface defined by the mounting boss. For example, once the second plurality of threads of assembly tool are at least partially received within the first plurality of threads of the mounting boss, a surgeon can manually rotate a T-handle, or otherwise operate a rotary power tool engaged with the shaft portion of the assembly tool, to cause the second plurality of threads to rotate relative to the first plurality of threads. In turn, a flange of the assembly tool can contact and engage the second component to cause the first coupling surface to slideably engage the outer coupling surface of the mounting boss in a controlled manner.

The foregoing systems and devices, etc. are merely illustrative of the components, interconnections, communications, functions, etc. that can be employed in carrying out examples in accordance with this disclosure. Different types and combinations of sensor or other portable electronics devices, computers including clients and servers, implants, and other systems and devices can be employed in examples according to this disclosure.

While the above description of the modular implant system is generally discussed with reference to the humerus in the context of shoulder arthroplasties, it to be appreciated that the modular implant system of the present disclosure, and any of its associated benefits, is also applicable to other ball and socket type joints, such as, but not limited to, the femur in the context of hip arthroplasties, or one or more bones of a wrist or elbow joint.

The above detailed description includes references to the accompanying drawings, which form a part of the detailed description. The drawings show, by way of illustration, specific embodiments in which the invention can be practiced. These embodiments are also referred to herein as "examples." Such examples can include elements in addition to those shown or described. However, the present inventor also contemplates examples in which only those elements shown or described are provided.

Moreover, the present inventor also contemplates examples using any combination or permutation of those elements shown or described (or one or more aspects thereof), either with respect to a particular example (or one or more aspects thereof), or with respect to other examples (or one or more aspects thereof) shown or described herein. In the event of inconsistent usages between this document and any documents so incorporated by reference, the usage in this document controls.

In this document, the terms "a" or "an" are used, as is common in patent documents, to include one or more than one, independent of any other instances or usages of "at least one" or "one or more." In this document, the term "or" is used to refer to a nonexclusive or, such that "A or B" includes "A but not B," "B but not A," and "A and B," unless otherwise indicated. In this document, the terms "including" and "in which" are used as the plain-English equivalents of the respective terms "comprising" and "wherein." Also, in the following claims, the terms "including" and "comprising" are open-ended, that is, a system, device, article, composition, formulation, or process that includes elements in addition to those listed after such a term in a claim are still deemed to fall within the scope of that claim. Moreover, in the following claims, the terms "first," "second," and "third," etc. are used merely as labels, and are not intended to impose numerical requirements on their objects.

The above description is intended to be illustrative, and not restrictive. For example, the above-described examples (or one or more aspects thereof) may be used in combination with each other. Other embodiments can be used, such as by one of ordinary skill in the art upon reviewing the above description. The Abstract is provided to comply with 37 C.F.R. § 1.72(b), to allow the reader to quickly ascertain the nature of the technical disclosure. It is submitted with the understanding that it will not be used to interpret or limit the scope or meaning of the claims. Also, in the above Detailed Description, various features may be grouped together to streamline the disclosure.

This should not be interpreted as intending that an unclaimed disclosed feature is essential to any claim. Rather, inventive subject matter may lie in less than all features of a particular disclosed embodiment. Thus, the following claims are hereby incorporated into the Detailed Description as examples or embodiments, with each claim standing on its own as a separate embodiment, and it is contemplated that such embodiments can be combined with each other in various combinations or permutations. The scope of the invention should be determined with reference to the appended claims, along with the full scope of equivalents to which such claims are entitled.

### EXAMPLES

The following, non-limiting examples, detail certain aspects of the present subject matter to solve the challenges and provide the benefits discussed herein, among others

Example 1 is a modular implant system, comprising: a first component including: a stem including a proximal portion and a distal portion, the distal portion adapted to be anchored within a medullary canal of a bone; and a mounting boss extending from the proximal portion, the mounting boss defining an outer coupling surface and an inner coupling surface; and a second component including a collar defining a first coupling surface, the first coupling surface adapted to engage the outer coupling surface of the mounting boss of the stem to secure the second component to the first component.

In Example 2, the subject matter of Example 1 includes, wherein the outer coupling surface of the mounting boss and the first coupling surface of the collar are configured to engage each other via a press fit or a taper fit.

In Example 3, the subject matter of Examples 1-2 includes, wherein the second component includes at least one fin extending radially outward from the collar.

In Example 4, the subject matter of Example 3 includes, wherein the at least one fin defines a plurality of first apertures, the plurality of first apertures adapted to receive a suture there through to secure one or more bone fragments to the second component.

In Example 5, the subject matter of Example 4 includes, wherein the collar includes a first collar and a second collar; and wherein the first collar and the second collar each include at least one planar fin of the at least one fin.

In Example 6, the subject matter of Examples 4-5 includes, wherein the outer coupling surface of the mounting boss defines a projection, and the first coupling surface of the collar defines a plurality of slots forming a radial arrangement; and wherein each slot of the plurality of slots is adapted to receive the projection to enable a user to selectively align the collar on the mounting boss.

In Example 7, the subject matter of Example 6 includes, wherein the second component, the proximal portion of the stem, and the distal portion of the stem each include a porous surface.

In Example 8, the subject matter of Examples 1-7 includes, wherein the second component includes a plurality of radial fins extending radially outward from the collar, the plurality of radial fins adapted to engage cancellous bone within the bone or fill an anatomical void defined at least partially within a metaphysis of the bone.

In Example 9, the subject matter of Example 8 includes, wherein the plurality of radial fins forms an asymmetric arrangement; and wherein the plurality of radial fins is based on a statistical bone model or on patient specific imaging data.

In Example 10, the subject matter of Example 9 includes, wherein each fin of the plurality of radial fins defines a first radial fin surface extending parallel to and laterally offset from a first axis defined by the stem, when the second component is secured to the first component.

In Example 11, the subject matter of Example 10 includes, wherein the second component and the proximal portion of the stem includes a porous surface.

In Example 12, the subject matter of Examples 1-11 includes, wherein the second component includes: a head portion adapted to fill an anatomical void within a patient, the head portion based on a statistical bone model or on patient specific imaging data; and a plurality of longitudinal fins extending longitudinally outward from a planar surface of the head portion.

In Example 13, the subject matter of Example 12 includes, wherein the head portion defines a lateral surface adapted to engage and support a patient's deltoid muscle.

In Example 14, the subject matter of Examples 12-13 includes, wherein each fin of the plurality of longitudinal fins extends parallel to and laterally offset from a first axis defined by the stem when the second component is secured to the first component; and wherein the plurality of longitudinal fins is adapted to extend into cancellous bone of a diaphysis of the bone.

In Example 15, the subject matter of Example 14 includes, wherein the plurality of longitudinal fins forms an asymmetric arrangement; and wherein the plurality of longitudinal fins is based on a statistical bone model or on patient specific imaging data.

In Example 16, the subject matter of Example 15 includes, wherein the head portion defines a plurality of passages adapted to receive a suture anchor therein or a suture there through to secure soft tissue to the second component.

In Example 17, the subject matter of Examples 1-16 includes, wherein the second component includes: a inner portion and an outer portion adapted to fill an anatomical void within a patient, the inner portion based on a statistical bone model or on patient specific imaging data; and a plurality of transverse passages adapted to receive a suture anchor therein or a suture there through to secure soft tissue to the second component.

Example 18 is a modular implant system comprising: a first component including: a stem including a proximal portion and a distal portion, the distal portion adapted to be anchored within a medullary canal of a bone; a mounting boss extending from the proximal portion, the mounting boss defining an outer coupling surface and an inner coupling surface; and a plurality of second components, wherein: each second component of the plurality of second components includes, a collar defining a first coupling surface, the first coupling surface adapted to engage the outer coupling surface of the mounting boss to secure any of the plurality of second components to the first component; and each second component of the plurality of second components is uniquely sized and shaped relative to other second components of the plurality of second components.

In Example 19, the subject matter of Example 18 includes, wherein at least one second component of the plurality of second components is adapted for a revision arthroplasty; and wherein at least one second component of the plurality of second components is adapted for a fracture correction arthroplasty.

In Example 20, the subject matter of Examples 18-19 includes, wherein at least one second component adapted for a shoulder revision arthroplasty includes a plurality of radial fins adapted to engage cancellous bone within the bone or fill an anatomical void defined at least partially within a metaphysis of the bone, the plurality of radial fins sized and shaped based on a statistical bone model or on patient specific imaging data; and wherein the at least one second component adapted for a fracture correction arthroplasty includes at least one fin extending radially outward from the collar, the at least one fin defining a plurality of first apertures adapted to receive a suture there through to secure one or more bone fragments to the second component.

In Example 21, the subject matter of Example 20 includes, wherein the at least one second component adapted for a revision arthroplasty includes a plurality of longitudinal fins adapted to extend into cancellous bone of a diaphysis of the bone, the plurality of longitudinal fins sized and shaped based on a statistical bone model or on patient specific imaging data; and wherein the at least one second component adapted for a fracture correction arthroplasty includes a central portion, a first end portion, and a second end portion each based on a statistical bone model or on patient specific imaging data.

In Example 22, the subject matter of Example 21 includes, wherein the at least one second component adapted for a fracture correction arthroplasty defines a plurality of second apertures, the plurality of second apertures adapted to receive an anchor therein or a suture there through to secure one or more bone fragments to an outer surface of the at least one second component.

In Example 23, the subject matter of Examples 18-22 includes, wherein the mounting boss defines a first plurality of threads adapted to threadedly engage a second plurality of threads defined by an assembly tool.

In Example 24, the subject matter of Examples 18-23 includes, wherein the modular implant system includes a plurality of first components wherein: the stem of each first component of the plurality of first components is uniquely sized and shaped relative to the stem of other first components of the plurality of first components; and the mounting boss of each first component of the plurality of first components is uniformly sized and shaped relative to the mounting boss of other first components of the plurality of first components.

Example 25 is a method of adapting a modular implant system to a patient, the method comprising: selecting a first component from a plurality of first components, the first component adapted to be anchored within a medullary canal of a bone of the patient; selecting a second component from a plurality of second components based on a physical characteristic of the bone, the second component adapted to engage at least one of a metaphysis or a diaphysis of the bone of the patient; and securing the second component to the first component.

In Example 26, the subject matter of Example 25 includes, wherein the method includes selectively positioning the second component on the first component.

In Example 27, the subject matter of Example 26 includes, wherein selecting the first component from the plurality of first components includes selecting the first component based on a longitudinal length of a porous surface of the first component.

In Example 28, the subject matter of Example 27 includes, wherein selecting the second component from the plurality of second components includes selecting the second component based on a number of bone fragments or an amount of the bone that can be preserved.

In Example 29, the subject matter of Examples 25-28 includes, wherein securing the second component includes inserting an assembly tool through the second component to threadedly engage a first plurality of threads defined within a mounting boss with a second plurality of threads defined by the assembly tool.

In Example 30, the subject matter of Example 29 includes, wherein securing the second component to the first component includes rotating the assembly tool to cause a first coupling surface defined by a collar of the second component to slidably engage an outer coupling surface defined by the mounting boss.

In Example 31, the subject matter of Examples 25-30 includes, wherein securing the second component to the first component includes cooling the first component to cause thermal contraction and heating the second component to cause thermal expansion.

Example 32 is at least one machine-readable medium including instructions that, when executed by processing circuitry, cause the processing circuitry to perform operations to implement of any of Examples 1-31.

Example 33 is an apparatus comprising means to implement of any of Examples 1-31.

Example 34 is a system to implement of any of Examples 1-31.

Example 35 is a method to implement of any of Examples 1-31.

## Claims

1. A modular implant system, comprising:
a first component including:
a stem including a proximal portion and a distal portion, the distal portion adapted to be anchored within a medullary canal of a bone; and
a mounting boss extending from the proximal portion, the mounting boss defining an outer coupling surface and an inner coupling surface; and
a second component including a collar defining a first coupling surface, the first coupling surface adapted to engage the outer coupling surface of the mounting boss of the stem to secure the second component to the first component.

2. The modular implant system of claim 1, wherein the outer coupling surface of the mounting boss and the first coupling surface of the collar are configured to engage each other via a press fit or a taper fit.

3. The modular implant system of claim 1 or claim 2, wherein the second component includes at least one fin extending radially outward from the collar.

4. The modular implant system of claim 3, wherein the at least one fin defines a plurality of first apertures, the plurality of first apertures adapted to receive a suture there through to secure one or more bone fragments to the second component.

5. The modular implant system of claim 4, wherein the collar includes a first collar and a second collar; and wherein the first collar and the second collar each include at least one planar fin of the at least one fin.

6. The modular implant system of claim 4, wherein the outer coupling surface of the mounting boss defines a projection, and the first coupling surface of the collar defines a plurality of slots forming a radial arrangement; and wherein each slot of the plurality of slots is adapted to receive the projection to enable a user to selectively align the collar on the mounting boss.

7. The modular implant system of any one of the preceding claims, wherein the second component, the proximal portion of the stem, and the distal portion of the stem each include a porous surface.

8. The modular implant system of claim 1, wherein the second component includes a plurality of radial fins extending radially outward from the collar, the plurality of radial fins adapted to engage cancellous bone within the bone or fill an anatomical void defined at least partially within a metaphysis of the bone.

9. The modular implant system of claim 8, wherein the plurality of radial fins forms an asymmetric arrangement; and wherein the plurality of radial fins is based on a statistical bone model or on patient specific imaging data.

10. The modular implant system of claim 9, wherein each fin of the plurality of radial fins defines a first radial fin surface extending parallel to and laterally offset from a first axis defined by the stem, when the second component is secured to the first component.

11. The modular implant system of any one of claims 8-10, wherein the second component and the proximal portion of the stem includes a porous surface.

12. The modular implant system of claim 1, wherein the second component includes:
a head portion adapted to fill an anatomical void within a patient, the head portion based on a statistical bone model or on patient specific imaging data; and
a plurality of longitudinal fins extending longitudinally outward from a planar surface of the head portion.

13. The modular implant system of claim 12, wherein the head portion defines a lateral surface adapted to engage and support a patient's deltoid muscle.

14. The modular implant system of claim 12 or claim 13, wherein each fin of the plurality of longitudinal fins extends parallel to and laterally offset from a first axis defined by the stem when the second component is secured to the first component; wherein the plurality of longitudinal fins is adapted to extend into cancellous bone of a diaphysis of the bone; wherein the plurality of longitudinal fins forms an asymmetric arrangement; and wherein the head portion defines a plurality of passages adapted to receive a suture anchor therein or a suture there through to secure soft tissue to the second component.

15. The modular implant system of any one of the preceding claims, comprising a plurality of the second components, wherein each second component of the plurality of second components is uniquely sized and shaped relative to other second components of the plurality of second components.
